# EUROPEAN PATENT APPLICATION

(11) **EP 3 315 967 A1**
(43) Date of publication of application: **02.05.2018**
(21) Application number: 16814726.2
(22) Date of filing: 24.06.2016
(51) Int. Cl.: G01N 33/53, C12Q 1/68, G01N 33/50

(54) **TM4SF19 AS MARKER FOR DIAGNOSING OBESITY AND METHOD USING SAME**

(30) Priority: 26.06.2015 KR 20150091189
(71) Applicant: Gilo Inc., Seoul 06671 (KR)
(72) Inventor: KIM, Seong Jin, Seoul 06667 (KR); PARK, Su Jin, Namyangju-si Gyeonggi-do 12151 (KR)
(74) Representative: Weickmann & Weickmann PartmbB
(86) International application number: PCT/KR2016/006731
(87) International publication number: WO 2016/209013

(57) **Abstract**

The diagnosis of obesity can be effectively carried out by using a composition for diagnosing obesity comprising a substance, which specifically couples to TM4SF19, a kit, and a method for diagnosing the obesity using the same. In addition, a candidate substance capable of treating obesity can be effectively screened in accordance with a method for screening a candidate substance for treating obesity, which inhibits the TM4SF19.

<110>    College of Medicine Pochon CHA University Industry-Academic
Cooperation Foundation

<120>    TM4SF19, a marker for diagnosing obesity, and methods using
         thereof

<130>    PX052356

<160>    40

<170>    KopatentIn 2.0

<210>    1
<211>    1073
<212>    RNA
<213>    Homo sapiens

<400>    1
acgtatatac agagcctccc tggccctcct ggaaagagtc ctggaaagac aaccttcagg        60

tccagccctg gagctggagg agtggagccc cactctgaag acgcagcctt tctccaggtt       120

ctgtctctcc cattctgatt cttgacacca gatgcaggat ggtgtcctct ccctgcacgc       180

aggcaagctc acggacttgc tcccgtatcc tgggactgag ccttgggact gcagccctgt       240

ttgctgctgg ggccaacgtg gcactcctcc ttcctaactg ggatgtcacc tacctgttga       300

ggggcctcct tggcaggcat gccatgctgg gaactgggct ctggggagga ggcctcatgg       360

tactcactgc agctatcctc atctccttga tgggctggag atacggctgc ttcagtaaga       420

gtgggctctg tcgaagcgtg cttactgctc tgttgtcagg tggcctggct ttacttggag       480

ccctgatttg ctttgtcact tctggagttg ctctgaaaga tggtcctttt tgcatgtttg       540

atgtttcatc cttcaatcag acacaagctt ggaaatatgg ttacccattc aaagacctgc       600

atagaattat ctgtatgacc gttcgctctg gaactccgtc tgcctggagc cctctgcagc       660

tgttgtctgg cacgtgtccc tcttctccgc ccttctgtgc atcagcctgc tccagcttct       720

cctggtggtc gttcatgtca tcaacagcct cctgggcctt ttctgcagcc tctgcgagaa       780

gtgacaggca gaaccttcac ttgcaagcat gggtgttttc atcatcggct gtcttgaatc       840

ctttctacaa ggagtgggta cgaattataa acaaacttcc cctttaggta tccctggagt       900

aataatgaca acaaaattca ctgcaggtcg gtggaatgat agaatgcatt ttaaatcaca       960

ttgtaaactt ccaggtgatc catggatagg ataaataact aagttattat aattgtttag      1020

gaatttatag tccataaaat atcctccagc cagggaaaaa aaaaaaaaaa aaa             1073


<210>    2
<211>    999
<212>    RNA
<213>    Homo sapiens

<400>    2
acgtatatac agagcctccc tggccctcct ggaaagagtc ctggaaagac aaccttcagg        60

tccagccctg gagctggagg agtggagccc cactctgaag acgcagcctt tctccaggtt 120

ctgtctctcc cattctgatt cttgacacca gatgcaggat ggtgtcctct ccctgcacgc 180

aggcaagctc acggacttgc tcccgtatcc tgggactgag ccttgggact gcagccctgt 240

ttgctgctgg ggccaacgtg gcactcctcc ttcctaactg ggatgtcacc tacctgttga 300

ggggcctcct tggcaggcat gccatgctgg gaactgggct ctggggagga ggcctcatgg 360

tgcttactgc tctgttgtca ggtggcctgg ctttacttgg agccctgatt tgctttgtca 420

cttctggagt tgctctgaaa gatggtcctt tttgcatgtt tgatgtttca tccttcaatc 480

agacacaagc ttggaaatat ggttacccat tcaaagacct gcatagtagg aattatctgt 540

atgaccgttc gctctggaac tccgtctgcc tggagccctc tgcagctgtt gtctggcacg 600

tgtccctctt ctccgccctt ctgtgcatca gcctgctcca gcttctcctg gtggtcgttc 660

atgtcatcaa cagcctcctg ggccttttct gcagcctctg cgagaagtga caggcagaac 720

cttcacttgc aagcatgggt gttttcatca tcggctgtct tgaatccttt ctacaaggag 780

tgggtacgaa ttataaacaa acttcccctt taggtatccc tggagtaata atgacaacaa 840

aattcactgc aggtcggtgg aatgatagaa tgcattttaa atcacattgt aaacttccag 900

gtgatccatg gataggataa ataactaagt tattataatt gtttaggaat ttatagtcca 960

taaaatatcc tccagccagg gaaaaaaaaa aaaaaaaaa 999

<210> 3
<211> 1077
<212> RNA
<213> Homo sapiens

<400> 3
   acgtatatac agagcctccc tggccctcct ggaaagagtc ctggaaagac aaccttcagg 60
   tccagccctg gagctggagg agtggagccc cactctgaag acgcagcctt tctccaggtt 120
   ctgtctctcc cattctgatt cttgacacca gatgcaggat ggtgtcctct ccctgcacgc 180
   aggcaagctc acggacttgc tcccgtatcc tgggactgag ccttgggact gcagccctgt 240
   ttgctgctgg ggccaacgtg gcactcctcc ttcctaactg ggatgtcacc tacctgttga 300
   ggggcctcct tggcaggcat gccatgctgg gaactgggct ctggggagga ggcctcatgg 360
   tactcactgc agctatcctc atctccttga tgggctggag atacggctgc ttcagtaaga 420
   gtgggctctg tcgaagcgtg cttactgctc tgttgtcagg tggcctggct ttacttggag 480
   ccctgatttg ctttgtcact tctggagttg ctctgaaaga tggtcctttt tgcatgtttg 540
   atgtttcatc cttcaatcag acacaagctt ggaaatatgg ttacccattc aaagacctgc 600
   atagtaggaa ttatctgtat gaccgttcgc tctggaactc cgtctgcctg gagccctctg 660
   cagctgttgt ctggcacgtg tccctcttct ccgcccttct gtgcatcagc ctgctccagc 720
   ttctcctggt ggtcgttcat gtcatcaaca gcctcctggg ccttttctgc agcctctgcg 780
   agaagtgaca ggcagaacct tcacttgcaa gcatgggtgt tttcatcatc ggctgtcttg 840
   aatcctttct acaaggagtg ggtacgaatt ataaacaaac ttccccttta ggtatccctg 900
   gagtaataat gacaacaaaa ttcactgcag gtcggtggaa tgatagaatg cattttaaat 960
   cacattgtaa acttccaggt gatccatgga taggataaat aactaagtta ttataattgt 1020
   ttaggaattt atagtccata aaatatcctc cagccaggga aaaaaaaaaa aaaaaaa 1077

<210> 4
<211> 1257
<212> RNA
<213> Mus musculus

<400> 4
gagcccagat ggtacaagga aggatgacac agccggatgg agcagctgat gtggaaaggg 60

cgctggtggc agaggaggct gagggactgc tcctgggtgc atcagtgact cactaagacc 120

gttgagcttg tgagtccatg tgtccttcct tggaatcaca tgaaagtgag cagagaatga 180

gtgggtcacg agcacaggct tcgcctggga cagaatgatt tttcagtgga ggcccgtggg 240

cagctgtgga cacccacgcc cactcgagtt cacatgaata cccaggcaac cgctacatat 300

gcaggacctt cccgaccctg caagcctagc cggctggggc tgcccgaggg gccaaggtcc 360

attttgaaga gtgtctcctc cactttcttg ttctcctatc ctgattcttg ccatcacaaa 420

caggatgctg tccttttccc gtgtggtgaa ctgctcacgg acctgttctc gcttcctggg 480

actgagtctg gggaccgcat ccctgtgtgc tgctggtgcc aacatcgcgc tcctctttcc 540

taactgggat gtgacctacc tgatgagggg cctcattggc aagcatgcca tgctgggctc 600

tgggctctgg ggaggaggcc tcatggttct cctggcagcc accctcatct ccatgacagg 660

cagcttcagt aagagtgcac cctgcctgca ggtgctcatt gctcttttgt caagtggcct 720

ggctctgctt ggggctgtga tttgctttgt cacttctgga gtagccttga aagatggtcc 780

cttttgcatg tttgatgtct catccttcaa tcagacacaa gcttggaaat tcggctatcc 840

ctttaaagat ctacacaaca ggaattatct gtatgaccgt tcactttgga cctccgtttg 900

cctggagccc tctaaggctg tggtttggca cgtggccttc ttctccatcc tcctgtgtat 960

cagcctcctc cagcttctct tggtggccat ccatcttgtc aatagcatcc tcggcctgtt 1020

ctgcagcttc tgtgagaagc actaggtaac ggatgccctc tcgtgaattg gcgcgctcag 1080

gacaagcttc ccccaacctt ttctgcaaga ataggctgtg aatccccttt agaccttctt 1140

ataggatgac agggctgcct tgctcacagc aacaaagtca tgctgctgta taaatgatct 1200

cagttcaacc ccttaaaaac ctcataaaag ggctggagga atggctcaga ggttgag 1257

<210> 5

<211>    242
<212>    PRT
<213>    Homo sapiens

<400>    5
Met Val Ser Ser Pro Cys Thr Gln Ala Ser Ser Arg Thr Cys Ser Arg
1               5                   10                  15

Ile Leu Gly Leu Ser Leu Gly Thr Ala Ala Leu Phe Ala Ala Gly Ala
            20                  25                  30

Asn Val Ala Leu Leu Leu Pro Asn Trp Asp Val Thr Tyr Leu Leu Arg
        35                  40                  45

Gly Leu Leu Gly Arg His Ala Met Leu Gly Thr Gly Leu Trp Gly Gly
    50                  55                  60

Gly Leu Met Val Leu Thr Ala Ala Ile Leu Ile Ser Leu Met Gly Trp
65                  70                  75                  80

Arg Tyr Gly Cys Phe Ser Lys Ser Gly Leu Cys Arg Ser Val Leu Thr
                85                  90                  95

Ala Leu Leu Ser Gly Gly Leu Ala Leu Leu Gly Ala Leu Ile Cys Phe
            100                 105                 110

Val Thr Ser Gly Val Ala Leu Lys Asp Gly Pro Phe Cys Met Phe Asp
        115                 120                 125

Val Ser Ser Phe Asn Gln Thr Gln Ala Trp Lys Tyr Gly Tyr Pro Phe
    130                 135                 140

Lys Asp Leu His Arg Ile Ile Cys Met Thr Val Arg Ser Gly Thr Pro
145                 150                 155                 160

Ser Ala Trp Ser Pro Leu Gln Leu Leu Ser Gly Thr Cys Pro Ser Ser
                165                 170                 175

Pro Pro Phe Cys Ala Ser Ala Cys Ser Ser Phe Ser Trp Trp Ser Phe
            180                 185                 190

Met Ser Ser Thr Ala Ser Trp Ala Phe Ser Ala Ala Ser Ala Arg Ser
        195                 200                 205

Asp Arg Gln Asn Leu His Leu Gln Ala Trp Val Phe Ser Ser Ser Ala
    210                 215                 220

Val Leu Asn Pro Phe Tyr Lys Glu Trp Val Arg Ile Ile Asn Lys Leu
225                 230                 235                 240

Pro Leu



<210>    6
<211>    183
<212>    PRT
<213>    Homo sapiens

<400>    6
Met Val Ser Ser Pro Cys Thr Gln Ala Ser Ser Arg Thr Cys Ser Arg
1               5                   10                  15

Ile Leu Gly Leu Ser Leu Gly Thr Ala Ala Leu Phe Ala Ala Gly Ala
            20                  25                  30

Asn Val Ala Leu Leu Leu Pro Asn Trp Asp Val Thr Tyr Leu Leu Arg
        35                  40                  45

Gly Leu Leu Gly Arg His Ala Met Leu Gly Thr Gly Leu Trp Gly Gly
    50                  55                  60

Gly Leu Met Val Leu Thr Ala Leu Leu Ser Gly Gly Leu Ala Leu Leu
65                  70                  75                  80

Gly Ala Leu Ile Cys Phe Val Thr Ser Gly Val Ala Leu Lys Asp Gly
                85                  90                  95

Pro Phe Cys Met Phe Asp Val Ser Ser Phe Asn Gln Thr Gln Ala Trp
            100                 105                 110

Lys Tyr Gly Tyr Pro Phe Lys Asp Leu His Ser Arg Asn Tyr Leu Tyr
        115                 120                 125

Asp Arg Ser Leu Trp Asn Ser Val Cys Leu Glu Pro Ser Ala Ala Val
    130                 135                 140

Val Trp His Val Ser Leu Phe Ser Ala Leu Leu Cys Ile Ser Leu Leu
145                 150                 155                 160

Gln Leu Leu Leu Val Val Val His Val Ile Asn Ser Leu Leu Gly Leu
                165                 170                 175

Phe Cys Ser Leu Cys Glu Lys
            180

<210> 7
<211> 209
<212> PRT
<213> Homo sapiens

<400> 7
Met Val Ser Ser Pro Cys Thr Gln Ala Ser Ser Arg Thr Cys Ser Arg
1               5                   10                  15

Ile Leu Gly Leu Ser Leu Gly Thr Ala Ala Leu Phe Ala Ala Gly Ala
            20                  25                  30

Asn Val Ala Leu Leu Leu Pro Asn Trp Asp Val Thr Tyr Leu Leu Arg
        35                  40                  45

Gly Leu Leu Gly Arg His Ala Met Leu Gly Thr Gly Leu Trp Gly Gly
    50                  55                  60

Gly Leu Met Val Leu Thr Ala Ala Ile Leu Ile Ser Leu Met Gly Trp
65                  70                  75                  80

Arg Tyr Gly Cys Phe Ser Lys Ser Gly Leu Cys Arg Ser Val Leu Thr
                85                  90                  95

Ala Leu Leu Ser Gly Gly Leu Ala Leu Leu Gly Ala Leu Ile Cys Phe
            100                 105                 110

Val Thr Ser Gly Val Ala Leu Lys Asp Gly Pro Phe Cys Met Phe Asp
        115                 120                 125

Val Ser Ser Phe Asn Gln Thr Gln Ala Trp Lys Tyr Gly Tyr Pro Phe
    130                 135                 140

Lys Asp Leu His Ser Arg Asn Tyr Leu Tyr Asp Arg Ser Leu Trp Asn
145                 150                 155                 160

Ser Val Cys Leu Glu Pro Ser Ala Ala Val Val Trp His Val Ser Leu
                165                 170                 175

Phe Ser Ala Leu Leu Cys Ile Ser Leu Leu Gln Leu Leu Leu Val Val
            180                 185                 190

Val His Val Ile Asn Ser Leu Leu Gly Leu Phe Cys Ser Leu Cys Glu
        195                 200                 205

Lys

<210>    8
<211>    206
<212>    PRT
<213>    Mus musculus

<400>    8
Met Leu Ser Phe Ser Arg Val Val Asn Cys Ser Arg Thr Cys Ser Arg
  1               5                  10                  15

Phe Leu Gly Leu Ser Leu Gly Thr Ala Ser Leu Cys Ala Ala Gly Ala
            20                  25                  30

Asn Ile Ala Leu Leu Phe Pro Asn Trp Asp Val Thr Tyr Leu Met Arg
        35                  40                  45

Gly Leu Ile Gly Lys His Ala Met Leu Gly Ser Gly Leu Trp Gly Gly
    50                  55                  60

Gly Leu Met Val Leu Leu Ala Ala Thr Leu Ile Ser Met Thr Gly Ser
 65                  70                  75                  80

Phe Ser Lys Ser Ala Pro Cys Leu Gln Val Leu Ile Ala Leu Leu Ser
                85                  90                  95

Ser Gly Leu Ala Leu Leu Gly Ala Val Ile Cys Phe Val Thr Ser Gly
            100                 105                 110

Val Ala Leu Lys Asp Gly Pro Phe Cys Met Phe Asp Val Ser Ser Phe
        115                 120                 125

Asn Gln Thr Gln Ala Trp Lys Phe Gly Tyr Pro Phe Lys Asp Leu His
    130                 135                 140

Asn Arg Asn Tyr Leu Tyr Asp Arg Ser Leu Trp Thr Ser Val Cys Leu
145                 150                 155                 160

Glu Pro Ser Lys Ala Val Val Trp His Val Ala Phe Phe Ser Ile Leu
                165                 170                 175

Leu Cys Ile Ser Leu Leu Gln Leu Leu Leu Val Ala Ile His Leu Val
            180                 185                 190

Asn Ser Ile Leu Gly Leu Phe Cys Ser Phe Cys Glu Lys His

195                 200                 205

<210>    9
<211>    20
<212>    DNA
<213>    Artificial Sequence

<220>
<223>    mouse TM4SF19 forward primer

<400>    9
ggctgtgatt tgctttgtca                                                    20

<210>    10
<211>    20
<212>    DNA
<213>    Artificial Sequence

<220>
<223>    mouse TM4SF19 reverse primer

<400>    10
agctggagga ggctgataca                                                    20

<210>    11
<211>    20
<212>    DNA
<213>    Artificial Sequence

<220>
<223>    human TM4SF19 forward primer

<400>    11
tatcctggga ctgagccttg                                                    20

<210>    12
<211>    20
<212>    DNA
<213>    Artificial Sequence

<220>
<223>    human TM4SF19 reverse primer

<400>    12
cttcgacaga gcccactctt                                                    20

<210>    13
<211>    23
<212>    DNA
<213>    Artificial Sequence

<220>

<223>    C/EBP alpha forward primer

<400>    13
gtgtgcacgt ctatgctaaa cca                                              23

<210>    14
<211>    25
<212>    DNA
<213>    Artificial Sequence

<220>
<223>    C/EBP alpha reverse primer

<400>    14
gccgttagtg aagagtctca gtttg                                            25

<210>    15
<211>    20
<212>    DNA
<213>    Artificial Sequence

<220>
<223>    PPAR gamma forward primer

<400>    15
cgctgatgca ctgcctatga                                                  20

<210>    16
<211>    22
<212>    DNA
<213>    Artificial Sequence

<220>
<223>    PPAR gamma reverse primer

<400>    16
agaggtccac agagctgatt cc                                               22

<210>    17
<211>    30
<212>    DNA
<213>    Artificial Sequence

<220>
<223>    human TM4SF19 full forward primer

<400>    17
gatcggatcc gtgtcctctc cctgcacgca                                       30

<210>    18
<211>    32
<212>    DNA
<213>    Artificial Sequence

<220>
<223>    human TM4SF19 full reverse primer


<400>    18
gatcgcggcc gctcacttct cgcagaggct gc                                    32


<210>    19
<211>    30
<212>    DNA
<213>    Artificial Sequence

<220>
<223>    human TM4SF19 D1 forward primer


<400>    19
gatcggatcc gtgtcctctc cctgcacgca                                       30


<210>    20
<211>    34
<212>    DNA
<213>    Artificial Sequence

<220>
<223>    human TM4SF19 D1 reverse primer


<400>    20
gatcgcggcc gcctacctca acaggtaggt gaca                                  34


<210>    21
<211>    30
<212>    DNA
<213>    Artificial Sequence

<220>
<223>    human TM4SF19 D2 forward primer


<400>    21
gatcggatcc gtgtcctctc cctgcacgca                                       30


<210>    22
<211>    34
<212>    DNA
<213>    Artificial Sequence

<220>
<223>    human TM4SF19 D2 reverse primer


<400>    22
gatcgcggcc gcctaactga agcagccgta tctc                                  34


<210>    23
<211>    33

<212>    DNA
<213>    Artificial Sequence

<220>
<223>    human TM4SF19 D3 forward primer


<400>    23
gatcggatcc atgaagagtg ggctctgtcg aag                                    33


<210>    24
<211>    32
<212>    DNA
<213>    Artificial Sequence

<220>
<223>    human TM4SF19 D3 reverse primer


<400>    24
gatcgcggcc gctcacttct cgcagaggct gc                                     32


<210>    25
<211>    33
<212>    DNA
<213>    Artificial Sequence

<220>
<223>    human TM4SF19 D4 forward primer


<400>    25
gatcggatcc atgctgtatg accgttcgct ctg                                    33


<210>    26
<211>    32
<212>    DNA
<213>    Artificial Sequence

<220>
<223>    human TM4SF19 D4 reverse primer


<400>    26
gatcgcggcc gctcacttct cgcagaggct gc                                     32


<210>    27
<211>    21
<212>    RNA
<213>    Artificial Sequence

<220>
<223>    GRP78-siRNA-1


<400>    27
ccgtacattc aagttgatat t                                                 21

<210>    28
<211>    21
<212>    RNA
<213>    Artificial Sequence

<220>
<223>    GRP78-siRNA-1


<400>    28
aatatcaact tgaatgtacg g                                                21


<210>    29
<211>    21
<212>    RNA
<213>    Artificial Sequence

<220>
<223>    GRP78-siRNA-2


<400>    29
cccttacact tggtattgaa a                                                21


<210>    30
<211>    21
<212>    RNA
<213>    Artificial Sequence

<220>
<223>    GRP78-siRNA-2


<400>    30
tttcaatacc aagtgtaagg g                                                21


<210>    31
<211>    28
<212>    DNA
<213>    Artificial Sequence

<220>
<223>    mouse TM4SF19 promoter forward primer -1964~+397


<400>    31
gatcagatct ggacccagca ctctcctg                                         28


<210>    32
<211>    30
<212>    DNA
<213>    Artificial Sequence

<220>
<223>    mouse TM4SF19 promoter reverse primer -1964~+397


<400>    32
gatcaagctt aggagaacaa gaaagtggag                                       30

<210>	33
<211>	33
<212>	DNA
<213>	Artificial Sequence

<220>
<223>	mouse TM4SF19 promoter forward primer -802~+397


<400>	33
gatcagatct cactccactt ccagaatttg tga	33


<210>	34
<211>	30
<212>	DNA
<213>	Artificial Sequence

<220>
<223>	mouse TM4SF19 promoter reverse primer -802~+397


<400>	34
gatcaagctt aggagaacaa gaaagtggag	30


<210>	35
<211>	30
<212>	DNA
<213>	Artificial Sequence

<220>
<223>	mouse TM4SF19 promoter forward primer -350~+397


<400>	35
gatcagatct agaaattctc gggtattagt	30


<210>	36
<211>	30
<212>	DNA
<213>	Artificial Sequence

<220>
<223>	mouse TM4SF19 promoter reverse primer -350~+397


<400>	36
gatcaagctt aggagaacaa gaaagtggag	30


<210>	37
<211>	30
<212>	DNA
<213>	Artificial Sequence

<220>
<223>	mouse TM4SF19 promoter forward primer +173~+397

<400>    37
gatcagatct tcgcctggga cagaatgatt                                        30


<210>    38
<211>    30
<212>    DNA
<213>    Artificial Sequence

<220>
<223>    mouse TM4SF19 promoter reverse primer +173~+397


<400>    38
gatcaagctt aggagaacaa gaaagtggag                                        30


<210>    39
<211>    30
<212>    DNA
<213>    Artificial Sequence

<220>
<223>    mouse TM4SF19 promoter forward primer +273~+397


<400>    39
gatcagatct gcaggacctt cccgaccctg                                        30


<210>    40
<211>    30
<212>    DNA
<213>    Artificial Sequence

<220>
<223>    mouse TM4SF19 promoter reverse primer +273~+397


<400>    40
gatcaagctt aggagaacaa gaaagtggag                                        30

## Description

### TECHNICAL FIELD

The present disclosure relates to a composition and a kit for diagnosing obesity including a substance specifically binding to transmembrane 4 L six family member 19 (TM4SF19), a method of diagnosing obesity by using the same, and a method of screening for a substance inhibiting TM4SF19.

### BACKGROUND ART

Obesity refers to a medical condition in which excess body fat has accumulated to such an extent that it may have a negative effect on health. Obesity is very closely related to occurrence of various adult diseases, and therefore, the higher the obesity, the greater the prevalence of diverse diseases, including diabetes, cholelithiasis, hypertension, heart disease, and stroke.

Obesity is caused by a combination of genetic, cultural, environmental factors, etc. In particular, the prevalence of obesity caused by excessive caloric intake or high fat diet is increasing in modern times.

Obesity may be diagnosed by various measures such as body mass index (BMI), body fat percentage, waist circumference, etc. However, these diagnostic methods depend only on limited physical characteristics. Therefore, it is difficult to accurately diagnose obesity only by these measures. Accordingly, it is necessary to develop a biomarker which may be used in the diagnosis of obesity and a method capable of diagnosing obesity with high accuracy by using the biomarker, based on biological mechanisms that cause obesity.

### DETAILED DESCRIPTION OF THE INVENTION

### TECHNICAL PROBLEM

An aspect provides a composition and a kit for diagnosing obesity including a substance specifically binding to transmembrane 4 L six family member 19 (TM4SF19).

Another aspect provides a method of measuring an expression level of TM4SF19 for diagnosing obesity of a subject.

Still another aspect provides a method of screening for a candidate for treating obesity, which inhibits TM4SF19.

### TECHNICAL SOLUTION

An aspect provides a composition for diagnosing obesity, including a substance capable of measuring an expression level of transmembrane 4 L six family member 19 (TM4SF19).

The TM4SF19 protein is a human (Homo sapiens)- or mouse (Mus musculus)-derived protein, but the same protein may be expressed in other mammals such as monkey, cow, horse, etc. The human-derived TM4SF19 may have three or more isoforms. The TM4SF19 may be peptides or proteins including amino acid sequences represented by NP_001191826.1 (SEQ ID NO: 5), NP_001191827.1 (SEQ ID NO: 6), NP_612470.2 (SEQ ID NO: 7), NP_001153874.1 (SEQ ID NO: 8) which are translated from mRNAs including GenBank Accession No. NM_001204897.1 (SEQ ID NO: 1), NM_001204898.1 (SEQ ID NO: 2), NM_138461.3 (SEQ ID NO: 3), and NM_001160402.1 (SEQ ID NO: 4), respectively. Nucleotide sequences or amino acid sequences which have biologically equivalent activity even though parts of the sequences are not identical to the mRNAs of SEQ ID NOS: 1 to 4 and the proteins of SEQ ID NOS: 5 to 8 may be regarded as TM4SF19 mRNA or TM4SF19 protein.

A nucleotide encoding TM4SF19 may have a nucleotide sequence having 60% or more, for example, 70% or more, 80% or more, 90% or more, 95% or more, 99% or more, or 100% sequence identity to any one of SEQ ID NOS: 1 to 4. Further, the nucleotide encoding TM4SF19 may have a nucleotide sequence including 1 or more different nucleotides, 2 or more different nucleotides, 3 or more different nucleotides, 4 or more different nucleotides, 5 or more different nucleotides, 6 or more different nucleotides, or 7 or more different nucleotides from any one nucleotide sequence of SEQ ID NOS: 1 to 4.

The TM4SF19 protein may include an amino acid sequence having 60% or more, for example, 70% or more, 80% or more, 90% or more, 95% or more, 99% or more, or 100% sequence identity to any one of SEQ ID NOS: 5 to 8. Further, the TM4SF19 protein may be a nucleotide having a sequence including 1 or more different nucleotides, 2 or more different nucleotides, 3 or more different nucleotides, 4 or more different nucleotides, 5 or more different nucleotides, 6 or more different nucleotides, or 7 or more different nucleotides from any one nucleotide sequence of SEQ ID NOS: 5 to 8.

The term "obesity" refers to a condition in which excess body fat has accumulated to such an extent that it may have a negative effect on health. Obesity may be diagnosed when BMI, waist circumference, or body fat percentage of a subject or a TM4SF19 expression level of the subject is higher than that of a control group. Obesity may be caused by diverse factors such as eating habits such as overeating or high fat diets, genetic factors, other diseases, infection, lack of exercise, or social factors.

The substance capable of measuring the TM4SF19 expression level may be an antibody, an antigen-binding fragment, a polypeptide, or a protein which specifically binds to the TM4SF19 protein or a fragment thereof, or a combination thereof.

The term "antibody" refers to a specific immunoglobulin directed against an antigenic site. The antibody refers to an antibody specifically binding to TM4SF19 which is a diagnostic marker for obesity. The antibody may be prepared from TM4SF19 protein according to a method commonly used in the art, after obtaining the TM4SF19 protein encoded by TM4SF19 gene by cloning the TM4SF19 gene into an expression vector. A type of the antibody includes a polyclonal antibody or a monoclonal antibody, and includes all immunoglobulin antibodies. The antibody includes not only complete forms having two full-length light chains and two full-length heavy chains but also functional fragments of antibody molecules which have a specific antigen binding site (binding domain) directed against an antigenic site to retain an antigen-binding function, although they do not have the intact complete antibody structure having two light chains and two heavy chains.

The substance capable of measuring the TM4SF19 expression level may be a probe, a primer, or a nucleotide sequence specifically binding to the nucleotide sequence encoding TM4SF19, or a combination thereof.

The nucleotide sequence of the TM4SF19 gene is known, and therefore, based on the sequence, those skilled in the art may design the primer or probe specifically binding to the nucleotide sequence according to a common method in the art.

The term "probe" refers to a nucleotide fragment such as RNA or DNA, which may specifically bind to a nucleotide such as mRNA and has a length of several bases to several hundred bases. The probe is labeled with a radioisotope so that the presence or absence, or the expression level of a specific mRNA may be determined. The probe may be constructed in the form of an oligonucleotide probe, a single stranded DNA probe, a double stranded DNA probe, an RNA probe, etc. According to a specific embodiment of the present invention, mRNA of TM4SF19 gene which is a marker for obesity and a complementary probe may be used to allow hybridization, and the mRNA expression level may be determined by the hybridization degree, thereby measuring the occurrence and degree of obesity. Appropriate probes and hybridization conditions may be properly selected according to a technology known in the art.

The term "primer" refers to a short nucleotide sequence having a free 3' hydroxyl group, which is able to undergo base-pairing interaction with a complementary template and serves as a starting point for replicating the template strand. A primer is able to initiate DNA synthesis in the presence of a reagent for polymerization (e.g., DNA polymerase or reverse transcriptase) and four different nucleoside triphosphates in suitable buffers and at a suitable temperature. According to a specific embodiment, PCR amplification may be performed by using a primer set of mRNA of the marker TM4SF19 to measure the expression level of the desired TM4SF19 protein, thereby diagnosing obesity. PCR conditions and a length of the primer set may be appropriately selected according to a technology known in the art.

The term "nucleotide" refers to deoxyribonucleotide or ribonucleotide, and unless otherwise mentioned, the nucleotide may include analogs of a natural nucleotide and analogs including modified sugars or bases.

The probe, primer, or nucleotide may be chemically synthesized using a phosphoramidite solid support method or other widely known methods. These nucleotide sequences may also be modified by using various methods known in the art. Examples of such modifications include methylation, capsulation, replacement of one or more native nucleotides with analogues thereof, and inter-nucleotide modifications, for example, modifications to uncharged conjugates (e.g., methyl phosphonate, phosphotriester, phosphoroamidate, carbamate, etc.) or charged conjugates (e.g., phosphorothioate, phosphorodithioate, etc.).

The probe, primer, or nucleotide may have a length of 10 nucleotides to 100 nucleotides (hereinafter, referred to as 'nt'), 10 nt to 90 nt, 10 nt to 80 nt, 10 nt to 70 nt, 10 nt to 60 nt, 10 nt to 50 nt, 10 nt to 40 nt, 10 nt to 30 nt, 10 nt to 25 nt, 20 nt to 100 nt, 30 nt to 90 nt, 40 nt to 80 nt, 50 nt to 70 nt, 20 nt to 60 nt, 20 nt to 50 nt, 30 nt to 40 nt, 20 nt to 30 nt, or 20 nt to 25 nt.

The expression level of TM4SF19 protein or the mRNA of the gene may be specifically increased in adipose tissues and preadipocytes of obese mice. Therefore, the composition for diagnosing obesity according to the present disclosure may be used to diagnose obesity by measuring the expression level of TM4SF19 gene in adipocytes or preadipocytes of a patient suspected of having obesity.

Another aspect provides a kit for diagnosing obesity, including the substance capable of measuring the expression level of TM4SF19.

The kit for diagnosing obesity may be used to diagnose obesity by determining the expression level of the TM4SF19 protein which is a diagnostic marker for obesity through measurement of the expression level of mRNA of the gene or the protein. The kit for diagnosing obesity may include the above-described substance capable of measuring the expression level of TM4SF19 protein, i.e., an antibody, an antigen binding fragment, a polypeptide, or a protein specifically binding to TM4SF19 protein, or a primer, a probe, or a nucleotide specifically binding to the gene encoding TM4SF19 protein, or a combination thereof, as well as a composition, solution or apparatus, which includes one or more kinds of different constituents suitable for analysis methods of measuring the expression level of TM4SF19 protein.

According to a specific embodiment, the kit for diagnosing obesity of the present disclosure may be a kit including essential elements required for performing RT-PCR when the kit is a kit for measuring the mRNA expression level of the TM4SF19 protein. An RT-PCR kit may further include test tubes or other suitable containers, reaction buffers, deoxynucleotides (dNTPs), enzymes such as Taq-polymerase and reverse transcriptase, DNAse, RNAse inhibitor, DEPC-treated water (dEPC-water), sterile water, etc., in addition to each primer pair specific to mRNA of the marker gene. Further, the kit may include a primer pair specific to a gene which is used as a quantitative control group.

According to another specific embodiment, the kit for diagnosing obesity of the present disclosure may include a matrix, an appropriate buffer solution, a coloring enzyme, or a secondary antibody labeled with a fluorescent substance, a coloring substrate, etc. for the immunological detection of the antibody, the antigen binding fragment, the polypeptide, or the protein antibody specifically binding to TM4SF19 protein. As for the matrix, a nitrocellulose membrane, a 96-well plate made of a polyvinyl resin, a 96-well plate made of a polystyrene resin, and a slide glass may be used. As for the coloring enzyme, peroxidase and alkaline phosphatase may be used. As for the fluorescent substance, FITC, RITC, etc. may be used, and as for the coloring substrate, 2,2'-azino-bis(3-ethylbenzthiazoline-6-sulfonic acid) (ABTS), o-phenylenediamine (OPD), tetramethyl benzidine (TMB), etc. may be used.

According to still another specific embodiment, the kit of the present disclosure may be a microarray for diagnosing obesity, which is able to measure the expression level of the protein or the expression level of mRNA of the gene encoding the protein. The microarray for diagnosing obesity may be easily prepared by using the marker of the present disclosure by those skilled in the art according to a method known in the art. According to a specific embodiment, the microarray may be a microarray in which mRNA of the gene encoding the TM4SF19 protein or cDNA of a sequence corresponding to a fragment thereof is attached as a probe to a substrate.

It is to be understood that among the terms or elements mentioned in the kit, those mentioned in the description of the claimed composition are as mentioned in the description of the composition claimed above.

Still another aspect provides a method of measuring the expression level of TM4SF19 for diagnosing obesity of a subject.

The method may include measuring the expression level of TM4SF19 by measuring an amount of a complex which is formed by contacting a sample separated from the subject with the substance specifically binding to TM4SF19.

The subject to be diagnosed may be a mammal. The mammal may be a human, a mouse, a rat, a cow, a goat, a pig, a horse, a sheep, a dog, a cat, or a combination thereof. The sample may include cells, preadipocytes, or adipocytes of the subject, or cells derived therefrom.

The measuring of the TM4SF19 expression level may be measuring of the expression level of TM4SF19 protein or measuring of the mRNA level of TM4SF19.

The measuring of the expression level of TM4SF19 protein may be performed by examining the expression level of the protein using an antibody, an antigen binding fragment, a polypeptide, or a protein specifically binding to the protein. Analysis methods therefor may include Western blotting, an enzyme linked immunosorbent assay (ELISA), a radioimmunoassay (RIA), radialimmunodiffusion, Ouchterlony immunodiffusion, rocket immunoelectrophoresis, immunohistostaining, an immunoprecipitation assay, a complement fixation assay, FACS, a protein chip, etc. These analysis methods may be appropriately performed by those skilled in the art by using a known technology.

The mRNA expression level of TM4SF19 may be measured by using a probe, a primer, or a nucleotide specifically binding to the mRNA. Analysis methods therefor may include RT-PCR, an RNase protection assay (RPA), Northern blotting, a DNA chip, etc. These analysis methods may be appropriately performed by those skilled in the art by using a known technology.

According to the method, obesity may be diagnosed by comparing expression levels of TM4SF19 protein between a sample of a subject and a normal control group. Specifically, expression levels of TM4SF19 protein are measured in a sample of a patient suspected of having obesity and a normal control group, and compared with each other. The occurrence and degree of obesity may be determined by examining whether the expression level of TM4SF19 protein is increased in the sample of the subject.

It is to be understood that among the terms or elements mentioned in the method, those mentioned in the description of the claimed composition and kit are as mentioned in the description of the composition and kit claimed above.

Still another aspect provides a method of screening for a candidate for treating obesity, which inhibits TM4SF19.

According to embodiments of the present disclosure, it was confirmed that TM4SF19 protein binds to Grp78 which is known to be involved in obesity, leading to stimulation of preadipocyte differentiation and lipid accumulation. Therefore, a substance inhibiting expression of TM4SF19 or a substance inhibiting activity of TM4SF19 in preadipocytes or adipocytes may be a candidate effective for the treatment of obesity.

Grp78 is a mouse (Mus musculus)-derived protein, but the same protein may be expressed in other mammals such as human, monkey, cow, horse, etc. Grp78 is a protein including a sequence represented by Uniprot reference no. P20029. A protein including an amino acid sequence which has biologically equivalent activity even though parts of the sequence are not identical to the above sequence may be regarded as Grp78 protein.

The substance inhibiting expression of TM4SF19 may be a substance specifically binding to a promoter sequence of TM4SF19 to inhibit transcription or a substance, such as an antibody, a polypeptide, a protein, or a nucleotide, specifically binding to mRNA of TM4SF19 to inhibit translation.

The screening method may include contacting the test substance with a sample including a promoter nucleotide sequence of TM4SF19. The sample may include cells, adipocytes, or preadipocytes separated from a mammal, or cells derived therefrom.

The screening method may include measuring activity of the promoter of TM4SF19 in the sample to which the test substance is added. The promoter sequence of TM4SF19 may be a SREBP or PPARγ transcription factor-binding site within -3000 of the promoter of TM4SF19.

The screening method may include measuring activity of the promoter of TM4SF19 in the sample. The measuring of the activity of the promoter may be performed by those skilled in the art by using a known technology, in which a labeled gene is linked to the promoter of TM4SF19, and then expression of the linked labeled gene is measured.

When the activity of the promoter of TM4SF19 is reduced, compared to that of the control group, the test substance added to the sample may be determined as a candidate for treating obesity.

The screening method may include contacting the test substance with the sample including TM4SF19 protein or a fragment thereof or a nucleotide sequence encoding the same. The sample may include cells, adipocytes, or preadipocytes separated from a mammal, or cells derived therefrom.

The screening method may include selecting the test substance that forms a complex with the TM4SF19 protein or the fragment thereof or the nucleotide sequence encoding the same in the sample to which the test substance is added. Detection of the complex may be appropriately preformed by those skilled in the art by using a known technology.

Further, the substance inhibiting activity of TM4SF19 may be a substance inhibiting binding of TM4SF19 and Grp78. The substance inhibiting binding of TM4SF19 and Grp78 may be a substance such as an antibody, a polypeptide, a protein, a nucleotide, etc. The substance may be a substance binding to a binding site of TM4SF19 and Grp78 or a transmembrane domain d of TM4SF19 and/or a linker region thereof.

It is to be understood that among the terms or elements mentioned in the method, those mentioned in the description of the claimed composition, kit, and diagnostic method are as mentioned in the description of the composition, kit, and diagnostic method claimed above.

Still another aspect provides a pharmaceutical composition for treating obesity, which inhibits TM4SF19, and a method of treating obesity by using the same.

Further, according to embodiments of the present invention, it was confirmed that TM4SF19 protein binds to Grp78 which is known to be involved in obesity, leading to stimulation of preadipocyte differentiation and lipid accumulation. Therefore, a substance inhibiting expression of TM4SF19 or a substance inhibiting activity of TM4SF19 in preadipocytes or adipocytes may be effective for the prevention and treatment of obesity.

It is to be understood that among the terms or elements mentioned in the composition and method, those mentioned in the description of the claimed composition, kit, diagnostic method, and screening method are as mentioned in the description of the composition, kit, diagnostic method, and screening method claimed above.

### ADVANTAGEOUS EFFECTS OF THE INVENTION

A composition and a kit for diagnosing obesity including a substance specifically binding to TM4SF19 according to an aspect may be effectively used in the diagnosis of obesity.

A method of measuring an expression level of TM4SF19 for diagnosing obesity of a subject according to another aspect may be effectively used in the diagnosis of obesity.

A method of screening for a candidate for treating obesity which inhibits TM4SF19 according to still another aspect may be used to effectively select the candidate capable of treating obesity.

### DESCRIPTION OF THE DRAWINGS

FIG. 1A is a graph showing the result of mRNA sequencing for analyzing TM4SF19 mRNA expression levels according to diet, and FIG. 1B shows the result of RT-PCR for measuring TM4SF19 mRNA expression levels according to diet;
FIG. 2 is a schematic view showing culture and differentiation model of preadipocytes;
FIG. 3 is a schematic view showing increased expression levels of TM4SF19 during adipocyte differentiation;
FIG. 4A is an image showing a comparison of lipid accumulation over time between TM4SF19-overexpressing preadipocyte and a control group, and FIG. 4B shows quantification of lipid accumulation over time in TM4SF19-overexpressing preadipocyte and the control group by measuring optical density at OD₄₂₀;
FIG. 5A is a graph showing increased mRNA expression of an adipogenic marker C/EBPα by TM4SF19 overexpression, and FIG. 5B is a graph showing increased mRNA expression of an adipogenic marker PPARγ by TM4SF19 overexpression;
FIG. 6 shows expression levels of adipogenic markers of TM4SF19-overexpressing cell line, as measured at a protein level;
FIG. 7 shows immunoprecipitation of proteins binding to TM4SF19 before and after preadipocyte differentiation;
FIGS. 8A and 8B are graphs showing relative expression levels of TM4SF19 mRNA and Grp78 mRNA according to diet, respectively;
FIG. 9A shows endogenous binding of TM4SF19 and Grp78 before and after adipocyte differentiation, and FIG. 9B shows binding of TM4SF19 and Grp78 overexpressed in 293T cells;
FIG. 10 is a schematic view showing deletion constructs of TM4SF19;
FIG. 11 shows whether deletion mutants of TM4SF19 bind with Grp78;
FIG. 12 shows decreased expressions of adipogenic markers by suppression of Grp78 expression in TM4SF19-overexpressing cell line;
FIG. 13 is a schematic view showing promoter deletion constructs of TM4SF19; and
FIG. 14 is a graph showing promoter activities of the prepared promoter deletion constructs of TM4SF19.

### MODE OF THE INVENTION

Hereinafter, the present invention will be described in more detail with reference to Examples. However, these Examples are for illustrative purposes only, and the scope of the present invention is not intended to be limited by these Examples.

### Example 1. Identification of TM4SF19 as obesity marker

### 1-1. Identification of TM4SF19 as obesity marker by using traditional natural product

A diet-induced obese animal model was administered with a traditional natural product which is known to have a therapeutic effect on obesity, and RNA sequencing analysis of an epididymal white adipose tissue thereof was performed to identify an obesity marker specifically reactive to the traditional natural product.

In detail, a feed to which Taeeumjowui-tang, green tea, or Bangpungtongseong-san was added was given to normal-diet or high-fat-diet C57BL/6J mice to develop animal models. Epididymal white adipose tissues of the mice were obtained, and 1 mL of TRIzol (Invitrogen, Grand Island, NY) per 0.1 g of the tissue was added to dissolve the tissues, and centrifugation was performed to obtain supernatants. 200 µL of chloroform was added to the obtained supernatants and centrifugation was performed for 20 minutes to obtain supernatants. An equal amount of isopropanol was added to the obtained supernatants, and centrifugation was performed for 10 minutes to obtain RNA pellets. The RNA pellets were washed with 75%(v/v) ethanol and dried, and then dissolved in diethylpyrocarbonate (DEPC)-treated water to prepare samples. mRNA sequencing analysis of the prepared samples was performed by Theragen Co., Ltd.

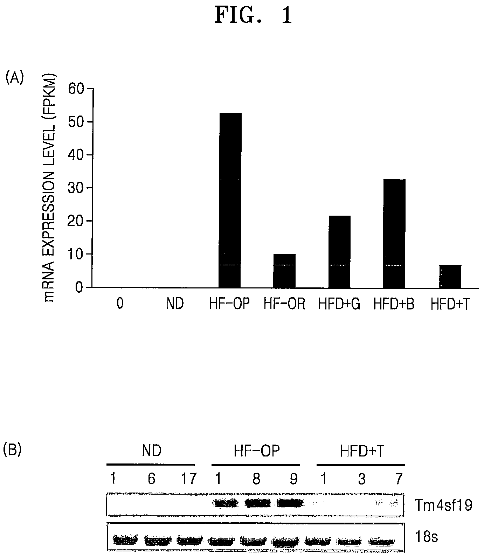

FIG. 1A shows TM4SF19 gene expression according to fragment per kb exon model (FPKM) in epididymal white adipose tissues of normal diet (ND) mice, high fat diet obesity-prone (HF-OP) mice, high fat diet obesity-resistant (HF-OR) mice, high fat diet+Taeeumjowui-tang (HFD+T)-fed mice, high fat diet+Bangpungtongseong-san(HFD+B)-fed mice, or high fat diet+green tea (HFD+G)-fed mice. As a result of the mRNA sequencing analysis, TM4SF19 mRNA was rarely expressed in the normal diet mice, but the expression levels thereof were greatly increased by high fat diet, and the expression was inhibited by the traditional natural product.

### 1-2. Examination of TM4SF19 mRNA expression level according to diet

Additionally, the TM4SF19 mRNA level of each subject was investigated again by PCR amplification. As shown in FIG. 1B, the TM4SF19 mRNA levels were increased by high fat diet, and decreased by Taeeumjowui-tang, as in the result of RNA sequencing. Primers used in PCR are the same as in the following Table 1.

**[Table 1]**

| | |
|---|---|
| Mouse TM4SF19 forward primer | GGCTGTGATTTGCTTTGTCA (SEQ ID NO: 9) |
| Mouse TM4SF19 reverse primer | AGCTGGAGGAGGCTGATACA (SEQ ID NO: 10) |

### Example 2. Examination of TM4SF19 expression level according to adipocyte differentiation

An effect of TM4SF19 on adipocyte differentiation was examined by using 3T3-L1 which is a preadipocyte.

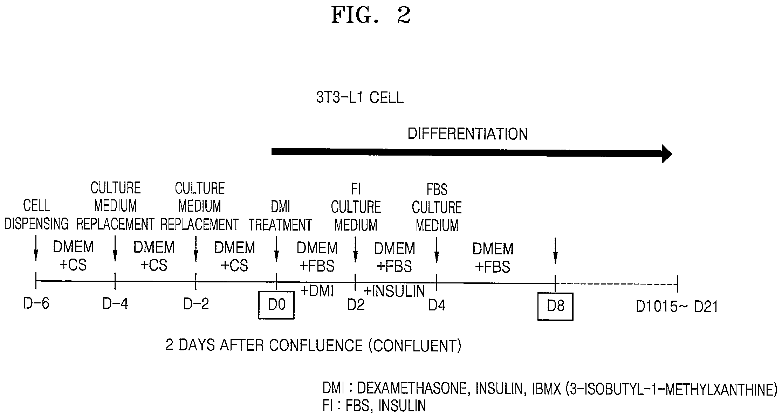

FIG. 2 is a schematic view showing culture and differentiation model of preadipocytes. The preadipocyte, 3T3-L1 was cultured in a DMEM medium supplemented with 10% bovine calf serum (BCS), 100 unit/mL penicillin, and 100 µg/mL streptomycin in a 5% CO₂ incubator at 37°C. To induce cell differentiation, cells were cultured in a DMEM medium containing 10%(v/v) FBS in the presence of DMI (Dexamethasone, Isobutylmethylxanthine (IBMX), and insulin), which is a differentiation inducer, for 2 days. 2 days later, the cell culture medium was replaced by a culture medium containing only insulin. 2 days later, the cell culture medium was replaced by a culture medium containing only 10%(v/v) FBS every other day, and then adipocytes were recovered. RNAs were obtained from the recovered adipocytes in the same manner as in Example 1. TM4SF19 expression levels were examined by RT-PCR using the primer set of Table 1.

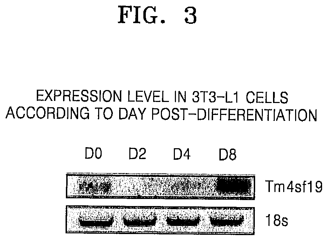

0, 2, 4, and 8 days after adding DMI, TM4SF19 expression levels were examined. As a result, it was confirmed that TM4SF19 mRNA levels were increased with progress of adipocyte differentiation, as shown in FIG. 3.

### Example 3. Examination of effect of TM4SF19 overexpression on adipocyte differentiation

### 3-1. Preparation of TM4SF19-overexpressing cell line

In order to examine the effect of TM4SF19 on adipocyte differentiation, TM4SF19 overexpression-induced 3T3-L1 cell line was established.

3HA-TM4SF19 having three hemagglutinin (HA) epitopes at the N-terminus was cloned into a viral vector LPCX (Laboratory of Cell Regulation and Carcinogenesis, NIH), and then the prepared viral vector was transfected into 3T3-L1 cells to establish a TM4SF19-overexpressing cell line. TM4SF19 mRNA overexpression was examined by RT-PCR. Primers used in PCR are as in the following Table 2.

**[Table 2]**

| | |
|---|---|
| Human TM4SF19 forward primer | TATCCTGGGACTGAGCCTTG (SEQ ID NO: 11) |
| Human TM4SF19 reverse primer | CTTCGACAGAGCCCACTCTT (SEQ ID NO: 12) |

### 3-2. Examination of lipid accumulation of adipocytes

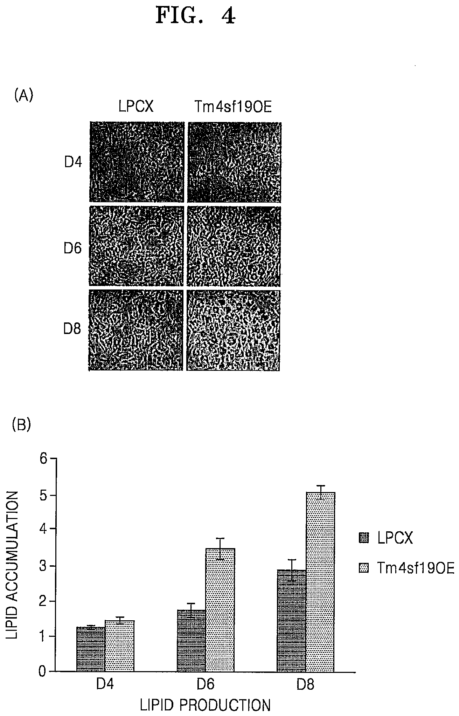

After inducing differentiation of 3T3-L1 cells by DMI, lipid droplet formation and lipid accumulation of adipocytes were examined by Oil Red O staining. FIG. 4A is an image showing lipid formation of adipocytes stained by Oil Red O staining, and FIG. 4B shows lipid accumulation of adipocytes. Isopropanol was added to stained adipocytes, and then incubated at room temperature. The adipocytes were transferred to a 96-well plate made of a polyvinyl resin and to a 96-well plate made of a polystyrene resin. Optical density was measured at OD₄₂₀, and quantified values were shown.

As shown in FIGS. 4A and 4B, it was confirmed that lipid accumulation was increased with progress of differentiation of TM4SF19-overexpressing preadipocytes into adipocytes, as compared with a control group.

### 3-3. Examination of expression levels of adipogenic markers of adipocytes

Next, after induction of adipocyte differentiation, mRNA levels of C/EBPα and PPARγ which are adipogenic markers were examined by real-time quantitative PCR. Primers used herein are as in the following Table 3.

**[Table 3]**

| | |
|---|---|
| C/EBPα forward primer | GTGTGCACGTCTATGCTAAACCA (SEQ ID NO: 13) |
| C/EBPα reverse primer | GCCGTTAGTGAAGAGTCTCAGTTTG (SEQ ID NO: 14) |
| PPARγ forward primer | CGCTGATGCACTGCCTATGA (SEQ ID NO: 15) |
| PPARγ reverse primer | AGAGGTCCACAGAGCTGATTCC (SEQ ID NO: 16) |

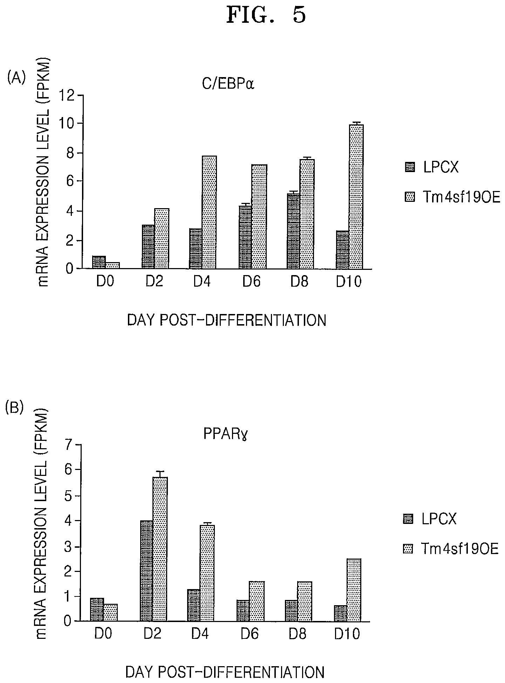

As a result, mRNA levels of C/EBPα and PPARγ in TM4SF19-overexpressing cells were remarkably increased, as compared with those of a control group, as shown in FIGS. 5A and 5B.

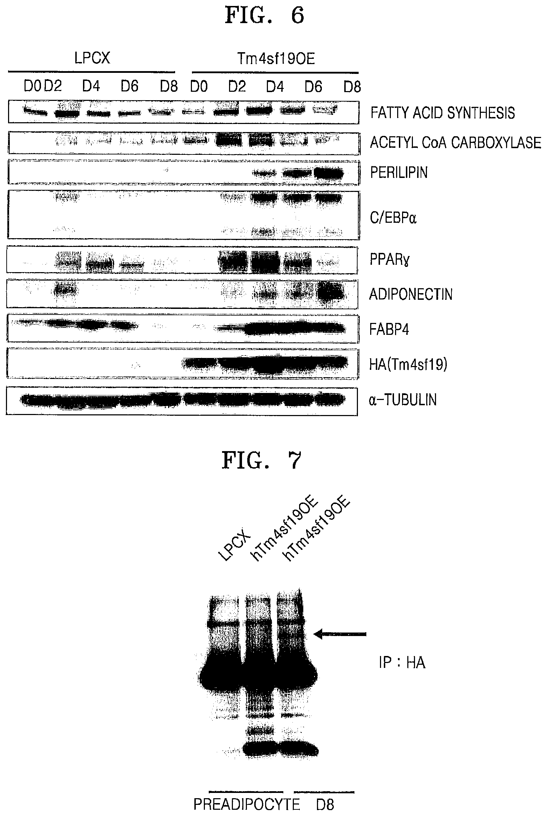

Further, expression levels of adipogenic markers were compared at a protein level. RIPA buffer was added to control LPCX and TM4SF19-overexpressing cell line, and then centrifugation was performed to obtain supernatants. Proteins were separated from the obtained supernatants, and electrophoresed on a SDS-PAGE gel. The proteins were transferred to PVDF, and antibodies against the adipogenic markers were attached to examine expression levels. 3HA-labeled TM4SF19 was examined by HA (Y-11 Santa Cruz Biotech), and the marker genes were examined by an adipogenic marker antibody sample kit (#12589, Cell Signaling). FIG. 6 shows expression levels of adipogenic markers in the TM4SF19-overexpressing cell line, as measured at a protein level.

As a result, expression levels of adiponectin, C/EBPα and PPARγ, fatty acid binding protein 4 (FABP4), fatty acid synthase, acetyl CoA carboxylase, and perilipin acting on formation of lipid droplet surface were remarkably increased in the TM4SF19-overexpressing cell line, as compared with the control group, as shown in FIG. 6. These results indicate that TM4SF19 acts on majors signaling involved in adipocyte differentiation.

### Example 4. Identification of Grp78 protein binding with Tm4f19

Since the role or mechanism of action of TM4SF19 has not been revealed, proteins binding to TM4SF19 before differentiation and at 8 days post-differentiation (D8) were examined by immunoprecipitation in order to identify proteins binding to TM4SF19 during adipocyte differentiation. As a result, a band indicating possibility of binding was detected, as shown in FIG. 7.

For additional analysis, the amount of TM4SF19-overexpressing cells was increased so that a sufficient amount of the protein was obtained at 8 days after adipocyte differentiation. Then, formaldehyde cross-linking and immunoprecipitation were performed. Immunoprecipitated proteins were stained with Coomassie blue and separated on a SDS-PAGE gel. The separated protein bands were cut and subjected to mass-spectrometry to analyze the proteins. Among many different candidate proteins thus analyzed, Grp78 (uniprot-MOUSE, P20029) which is known to be involved in obesity was selected and used in a subsequent experiment for examining differences of Grp78 expression according to diet.

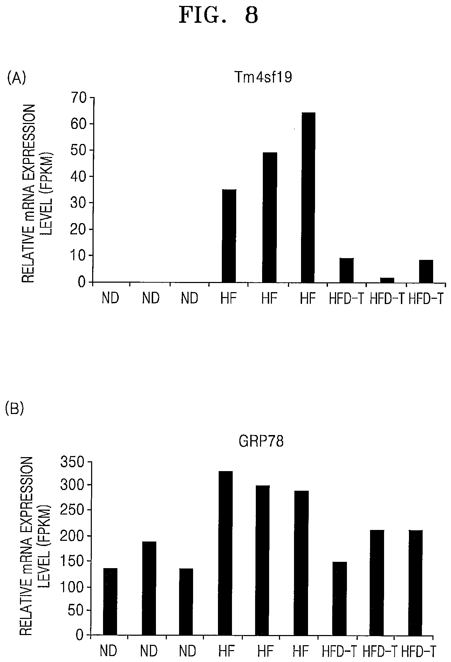

As a result, it was confirmed that TM4SF19 and Grp78 gene expression levels were increased in high fat diet (HF), as compared with normal diet (ND), and decreased by high fat diet+Taeeumjowui-tang (HFD-T), as shown in FIGS. 8A and 8B.

### Example 5. Examination of binding of TM4SF19 and Grp78

Endogenous binding of TM4SF19 and Grp78 before adipocyte differentiation (D0) and after adipocyte differentiation (D7) was examined.

In detail, to examine endogenous binding of TM4SF19 and Grp78, the proteins of TM4SF19-overexpressing cells were divided before and after differentiation, and immunoprecipitated with HA. The obtained proteins were immunoblotted by using Grp78 antibody (N-20, Santa Cruz Biotech). To examine *in vitro* binding of TM4SF19 and Grp78, 3HA-TM4SF19 and flag-Grp78 were transfected into 293T cells (ATCC) by using polyethyleneimine (PEI) as a carrier. Thereafter, immunoprecipitation was performed by using anti-Flag antibody (M2, Sigma), and the resulting product was identified by anti-HA antibody (Y-11 Santa Cruz Biotech).

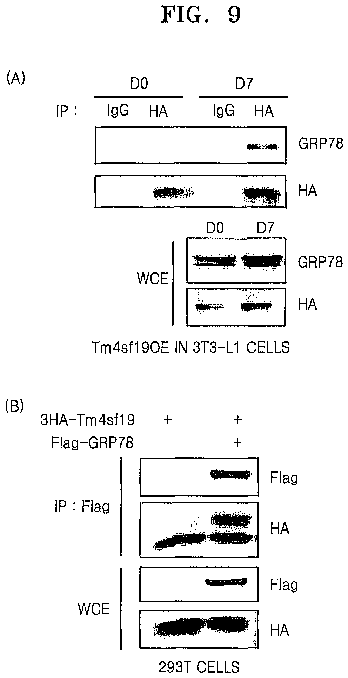

As a result, binding of the two proteins was observed after cell differentiation, as shown in FIG. 9A. After the two proteins were overexpressed in 293T, *in vitro* binding of the two proteins was examined. As a result, binding of the two proteins was observed, as shown in FIG. 9B.

### Example 6. Examination of binding site of TM4SF19 and Grp78

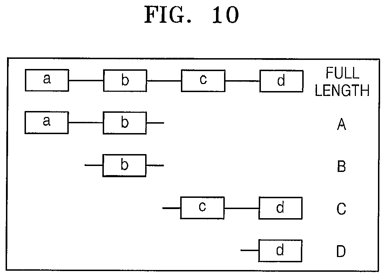

TM4SF19 consists of four transmembrane domains, and there are no known domains yet. On the basis of this, in order to examine a binding site of TM4SF19 and Grp78, four kinds (A, B, C and D) of TM4SF19 domain deletion mutants labeled with GST at the N-terminus were prepared, as shown in FIG. 10.

In detail, the desired TM4SF19 domain deletion mutants were prepared by polymerase chain reaction using 3HA-TM4SF19 as a template and primers of the following Table 4. The PCR product was cloned into a vector with an N-terminal GST tag (pGEX-2T, Smith and Johnson, Gene 67:31, (1988)) by using an appropriate restriction enzyme.

**[Table 4]**

| | | Sequence | Restriction enzyme |
|---|---|---|---|
| Human TM4SF19 full length | Forward | GATCGGATCCGTGTCCTCTCCCTGCACGCA (SEQ ID NO: 17) | BamHI |
| | Reverse | GATCGCGGCCGCTCACTTCTCGCAGAGGCTGC (SEQ ID NO: 18) | NotI |
| Human TM4SF19 D1 | Forward | GATCGGATCCGTGTCCTCTCCCTGCACGCA (SEQ ID NO: 19) | BamHI |
| | Reverse | GATCGCGGCCGCCTACCTCAACAGGTAGGTGACA (SEQ ID NO: 20) | NotI |
| Human TM4SF19 D2 | Forward | GATCGGATCCGTGTCCTCTCCCTGCACGCA (SEQ ID NO: 21) | BamHI |
| | Reverse | GATCGCGGCCGCCTAACTGAAGCAGCCGTATCTC (SEQ ID NO: 22) | NotI |
| Human TM4SF19 D3 | Forward | GATCGGATCCATGAAGAGTGGGCTCTGTCGAAG (SEQ ID NO: 23) | BamHI |
| | Reverse | GATCGCGGCCGCTCACTTCTCGCAGAGGCTGC (SEQ ID NO: 24) | NotI |
| Human TM4SF19 D4 | Forward | GATCGGATCCATGCTGTATGACCGTTCGCTCTG (SEQ ID NO: 25) | BamHI |
| | Reverse | GATCGCGGCCGCTCACTTCTCGCAGAGGCTGC (SEQ ID NO: 26) | NotI |

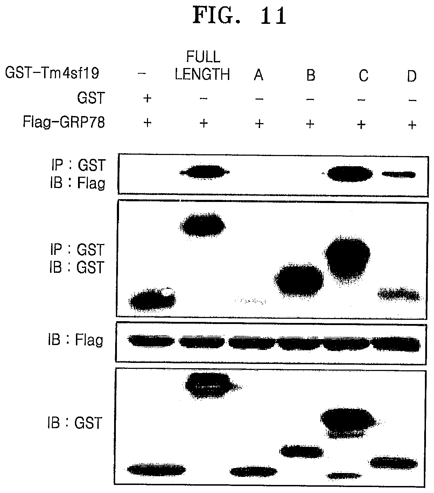

As a result, wild-type TM4SF19, and mutants C and D were found to bind with Grp78, as shown in FIG. 11, indicating that transmembrane domain d and a linker region thereof are essential for binding of the two proteins, TM4SF19 and Grp78.

### Example 7. Examination of adipocyte differentiation of TM4SF19 by Grp78

In order to investigate a mechanism whereby TM4SF19 overexpression increases adipocyte differentiation, mouse siRNAs of the following Table 5 were added to TM4SF19-overexpressing cell line to induce RNA interference, thereby suppressing Grp78 expression.

In detail, GRP78 siRNA and Lipofectamine RNAiMAX which is a transfection reagent manufactured by Invitrogen were reacted according to the manufacturer's recommendations to form a complex, which was added to TM4SF19-overexpressing NIH-3T3 L1 cell line, thereby inducing suppression of GRP78 expression by siRNA. Suppression of GRP78 expression was examined by real-time qPCR.

**[Table 5]**

| | |
|---|---|
| GRP78-siRNA-1 | CCGTACATTCAAGTTGATATT (SEQ ID NO: 27) |
| | AATATCAACTTGAATGTACGG (SEQ ID NO: 28) |
| GRP78-siRNA-2 | CCCTTACACTTGGTATTGAAA (SEQ ID NO: 29) |
| | TTTCAATACCAAGTGTAAGGG (SEQ ID NO: 30) |

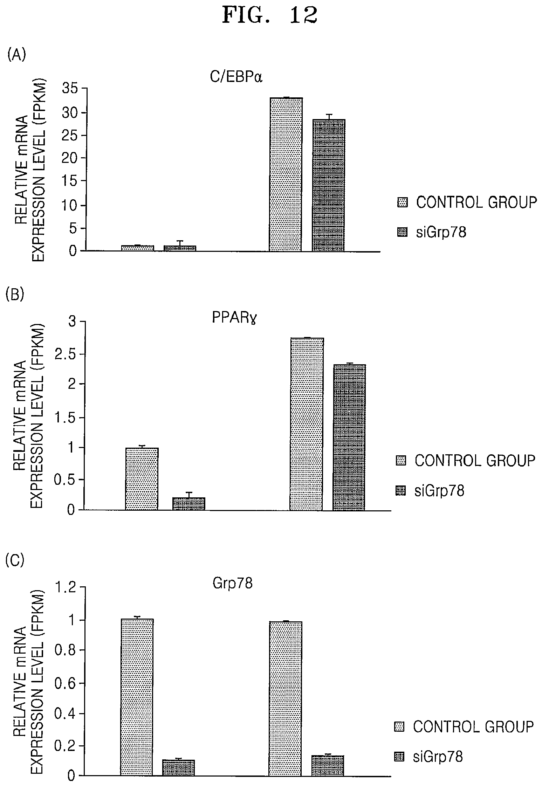

Adipocyte differentiation ability of the cell line was examined. As a result, adipocyte differentiation ability was reduced, as compared with a control group, as shown in FIG. 12. Therefore, it can be seen that TM4SF19 increases adipocyte differentiation via Grp78.

### Example 8. Examination of transcription factor binding site of TM4SF19 promoter

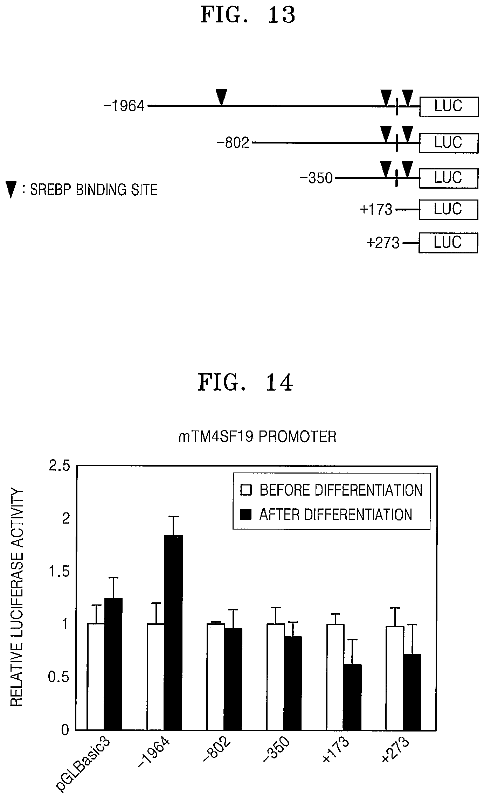

Three SREBP binding sites which are crucial for adipocyte differentiation are present at -1964 of the promoter of TM4SF19. In order to prepare deletion promoter constructs of TM4SF19 as in FIG. 13, genomic DNA (gDNA) was extracted from NIH-3T3 L1 preadipocytes by using a kit. Polymerase chain reaction (PCR) was performed by using primers of the following Table 6 to amplify the amounts of the desired deletion promoters of TM4SF19. A pGL3-basic vector (Promega) which is commonly used in promoter analysis and has luciferase was digested with an appropriate restriction enzyme and cloned at the end of the amplified deletion promoter of TM4SF19. Each of the deletion promoter constructs of TM4SF19 which were cloned for promoter analysis was transfected into NIH-3T3 L1 cells by using PEI, and adipocyte differentiation of NIH-3T3 L1 cells were induced. After completion of adipocyte differentiation, cells were lysed, and a substrate for luciferase was added to measure activity of luciferase, thereby measuring activity of the deletion promoter of TM4SF19.

**[Table 6]**

| Promoter region | | Sequence | Restriction enzyme |
|---|---|---|---|
| Mouse TM4SF19 -1964∼+397 | Forward | GATCAGATCTGGACCCAGCACTCTCCTG (SEQ ID NO: 31) | BglII |
| | Reverse | GATCAAGCTTAGGAGAACAAGAAAGTGGAG (SEQ ID NO: 32) | HindIII |
| Mouse TM4SF19 -802∼+397 | Forward | GATCAGATCTCACTCCACTTCCAGAATTTGTGA (SEQ ID NO: 33) | BglII |
| | Reverse | GATCAAGCTTAGGAGAACAAGAAAGTGGAG (SEQ ID NO: 34) | HindIII |
| Mouse TM4SF19 -350∼+397 | Forward | GATCAGATCTAGAAATTCTCGGGTATTAGT (SEQ ID NO: 35) | BglII |
| | Reverse | GATCAAGCTTAGGAGAACAAGAAAGTGGAG (SEQ ID NO: 36) | HindIII |
| Mouse TM4SF19 +173∼+397 | Forward | GATCAGATCTTCGCCTGGGACAGAATGATT (SEQ ID NO: 37) | BglII |
| | Reverse | GATCAAGCTTAGGAGAACAAGAAAGTGGAG (SEQ ID NO: 38) | HindIII |
| Mouse TM4SF19 +273∼+397 | Forward | GATCAGATCTGCAGGACCTTCCCGACCCTG (SEQ ID NO: 39) | BglII |
| | Reverse | GATCAAGCTTAGGAGAACAAGAAAGTGGAG (SEQ ID NO: 40) | HindIII |

As a result, there was a great difference in promoter activity according to the presence of a region from -1964 to -802, as shown in FIG. 14, indicating the promoter region plays an important role in adipocyte differentiation. Further, major transcription factors acting on the region were analyzed, and as a result, it was found that SREBP known to play an important role in adipocyte differentiation binds to the region.

Accordingly, it was confirmed that substances binding to the region from -1964 to -802 of the TM4SF19 promoter may be screened and developed as candidate therapeutic agents for obesity.

## Claims

1. A composition for diagnosing obesity, comprising an antibody, an antigen binding fragment, or a polypeptide specifically binding to a TM4SF19 protein or a fragment thereof, or a probe, a primer set, or a nucleotide specifically binding to a nucleotide sequence encoding the TM4SF19 protein.

2. The composition of claim 1, wherein the TM4SF19 protein comprises any one sequence of SEQ ID NOS: 5 to 8, and the nucleotide sequence encoding the TM4SF19 protein comprises any one sequence of SEQ ID NOS: 1 to 4.

3. The composition of claim 1, wherein the antibody, the antigen binding fragment, or the polypeptide binds to a transmembrane domain d of TM4SF19 and/or a linker region thereof.

4. A kit for diagnosing obesity, comprising the composition of any one of claims 1 to 3 and a reagent for detecting the same.

5. A method of measuring an expression level of TM4SF19 from a complex which is formed by contacting a sample separated from a subject with an antibody, a peptide, or a protein specifically binding to a TM4SF19 protein or a fragment thereof, or a combination thereof, or a probe, a primer, or a nucleotide specifically binding to a nucleotide sequence encoding the TM4SF19 protein, or a combination thereof.

6. The method of claim 5, wherein the measuring of the expression level is performed by any one or more methods selected from RT-PCR, RNase protection assay (RPA), Northern blotting, and a DNA chip.

7. The method of claim 5, wherein the measuring of the expression level is performed by any one or more methods selected from Western blotting, ELISA, a radioimmunoassay, radialimmunodiffusion, Ouchterlony immunodiffusion, rocket immunoelectrophoresis, immunohistostaining, an immunoprecipitation assay, a complement fixation assay, FACS, and a protein chip.

8. The method of claim 5, wherein the sample comprises preadipocytes or adipocytes of the subject.

9. A method of screening for a candidate for treating obesity, the method comprising:
contacting test substances with cells comprising a promoter nucleotide sequence of TM4SF19;
measuring activity of the promoter of TM4SF19 in the cells; and
selecting the test substance which decreases the activity of the promoter of TM4SF19, as compared with a control group.

10. The method of claim 9, wherein the promoter nucleotide sequence is a sequence at -802 to -1964 from the 5'-terminus of the sequence encoding the TM4SF19 protein.

11. A method of screening for a candidate for treating obesity, the method comprising:
contacting test substances with cells comprising a TM4SF19 protein or a fragment thereof, or a nucleotide sequence encoding the same; and
selecting the test substance which forms a complex with the TM4SF19 protein or the fragment thereof, or the nucleotide sequence encoding the same in the sample to inhibit function of the TM4SF19 protein or to suppress expression of the TM4SF19 protein.

12. The method of claim 11, wherein the test substance binds to a transmembrane domain d of TM4SF19 and/or a linker region thereof.

13. The method of claim 11, wherein the test substance inhibits binding of TM4SF19 protein and Grp78 protein.

14. The method of claim 11, wherein the test substance inhibits preadipocyte differentiation.
